# EUROPEAN PATENT SPECIFICATION

(11) **EP 1 011 507 B1**
(45) Date of publication and mention of the grant of the patent: **01.12.2004**
(21) Application number: 98901413.9
(22) Date of filing: 28.01.1998
(51) Int. Cl.: A61B 17/86

(54) **BONE FIXATION SYSTEM**
KNOCHEN-BEFESTIGUNGSSYSTEM
SYSTEME DE FIXATION D'OS

(30) Priority: 31.01.1997 GB 9701994; 24.03.1997 GB 9706216
(43) Date of publication of application: 28.06.2000
(73) Proprietor: Surgical Technology (UK) Limited, Bolton BL2 1DG (GB)
(72) Inventor: BROWN, Paul, Francis, Bolton BL2 6TW (GB); JONES, David, Carl, Liverpool L9 1AE (GB)
(74) Representative: Barker, Rosemary Anne
(86) International application number: PCT/GB1998/000261
(87) International publication number: WO 1998/033448

(56) References cited:
- EP-A- 0 498 709
- WO-A-93/12734
- GB-A- 2 269 753
- US-A- 3 675 327
- US-A- 4 869 242

## Description

This invention concerns a bone fixation system comprising a plurality of pegs adapted to be partially embedded in bone and providing means of securing one or more wires, spring coils, cords or elastics bands, or the like, so that the latter can be used to tie together two or more bones or parts of bones.

A system of this type for tying together a pair of adjacent vertebrae in the spine is disclosed in GB-A-2 269 753.

However, the present invention is applicable to intermaxillary or inter-dental fixation, where the upper and lower jaws of a patient are to be connected to each other and held at a fixed distance apart. Naturally, at least one such peg needs to be provided on each bone or part bone which is to be connected.

In the system of invention, each fixation peg comprises a shaft projecting from an enlarged head, the shaft being screw threaded and the head having top and bottom surfaces and an annular groove of concave cross-section in a region between said top and bottom surfaces.

Therefore, in use, with the screw threaded-shaft of at least one such peg embedded into respective bones or bone parts which are to be connected, wires, spring coils, cords or elastic bands can be entrained around the heads of these pegs, accommodated in the grooved areas thereof, so as to secure the. bones or bone parts together.

The preferred material of the pegs is titanium. However, stainless steel or other suitable metal or non-metal materials may also be used.

The invention will be described further, by way of example, with reference to the accompanying drawing, in which:
Fig. 1 is a side elevation of an intermaxillary fixation peg in accordance with the invention;
Fig. 2 is a plan view of the peg of Fig. 1;
Fig. 3. is a sketch showing four such pegs in use in the jaw area of a patient; and
Fig. 4 is a schematic section along line IV-IV in Fig. 3.

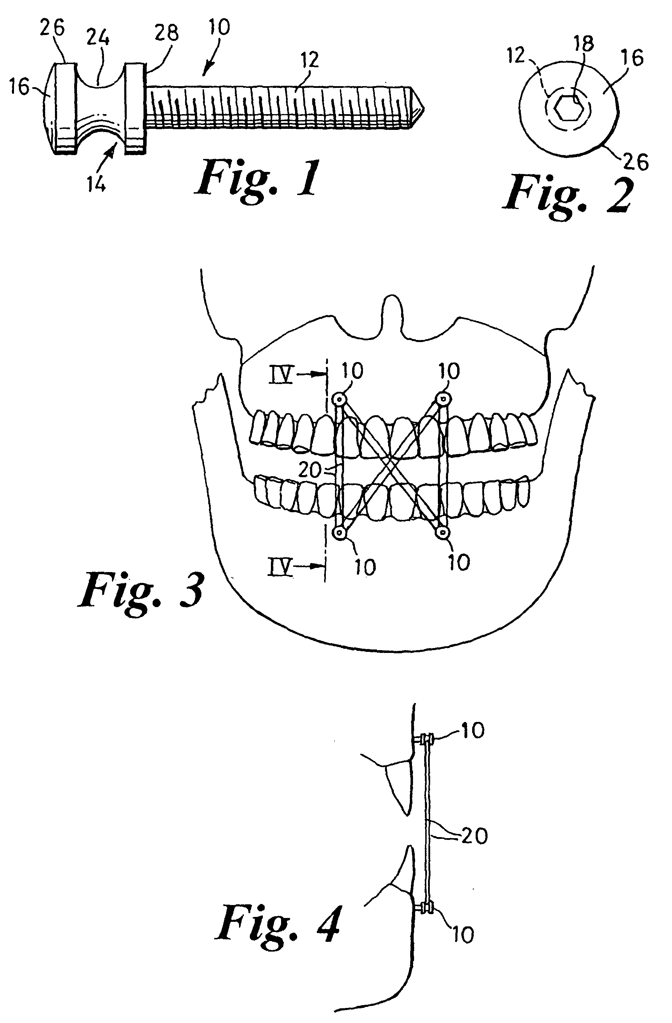

As shown in Figs. 1 and 2, an intermaxillary or interdental fixation peg (10) consists of a shaft (12) extending from an enlarged head (14). The shaft (12) is screw threaded and self tapping in that it will form its own threaded bore as it is screwed into bone. It is about 2mm in diameter and pointed at its end remote from the head (14). The shaft may be about 12mm long.

The head (14) is about 4mm in diameter and has a slightly convex upper surface (16), centrally of which there is a hexagonal recess (18) for reception of a correspondingly shaped head of a screw driver (not shown). The head (14) is also about 4mm high and an annular groove (24), which is of concave cross section and may be about 2mm wide, is formed in its central region, intermediate its upper surface (16) and its lower surface (28).

The peg (10) is made of titanium.

In use, as illustrated in Figs. 3 & 4, two such pegs (10) are screwed into each of the upper and lower jaw bones, using a hexagonal headed screwdriver engaging into the recess (18). Once these are suitably secured, wires (20) , or other similar elongate elements, are guided around the protruding heads (14), locating in the grooves (24) and are pulled as tight as is necessary and then secured by tying or twisting together or some other means. Because the wires (20) locate in the grooves (24), they are retained by the overhanging upper portions (26) of the heads (14). Therefore, there is no tendency for the wires (20) to slide off the heads of the pegs (10).

The foregoing is, of course, illustrative and not limitative of the scope of the invention. The dimensions are only given by way of example and may be varied in other embodiments. Also the material of the peg and the shape of any recess provided to allow tightening/loosening by means of a screwdriver may be varied from those mentioned in connection with the illustrated embodiment. Other variations in details are also possible.

## Claims

1. A bone fixation system comprising a plurality of fixation pegs (10), each comprising a shaft (12) projecting from an enlarged head (14), the shaft (12) being screw threaded and the head (14) having top and bottom surfaces (16, 28) and an annular groove (24) extending therearound in a region between said top and bottom surfaces, and a band, card or wire (20) which is adapted for entrainment around the grooves of respective pegs (10) to tie them together when, in use, one or more such pegs (10) are secured to each bone or bone part to be joined, by the screwing of the shaft (12) thereof into the bone or bone part, **characterised in that** the system is an intermaxillary fixation system and **in that** the head (14) of each peg (10) has only a single groove (24) of concave cross-section formed therein

2. An intermaxillary fixation system according to claim 1 wherein the head (14) of each peg (10) has a recess (18) in its top surface (16), which recess is adapted for reception of a screwdriver tip.

## Patentansprüche

1. Knochenfixierungssystem, mit einer Mehrzahl von Fixierungsstiften (10), deren jeder einen aus einem verbreiterten Kopf (14) vorstehenden Schaft (12) umfaßt, wobei der Schaft (12) mit einem Schraubgewinde versehen ist und der Kopf (14) eine Ober- und eine Unterseite (16, 28) sowie eine in einem Bereich zwischen der Ober- und Unterseite um diesen herumlaufende Ringnut (24) aufweist, und mit einem Band, einer Schnur oder einem Draht (20) zum Herumführen um die Nuten entsprechender Stifte (10), um sie zusammenzubinden, wenn einer oder mehrere solcher Stifte (10) bei Benutzung an jedem zusammenzufügenden Knochen oder Teil eines Knochens durch Einschrauben seines/ihres Schaftes (12) in den Knochen oder Teil eines Knochens befestigt wird/werden, **dadurch gekennzeichnet, daß** das System ein intermaxillares Befestigungssystem ist und daß der Kopf (14) jedes Stiftes (10) nur eine einzige, darin gebildete Nut (24) mit konkavem Querschnitt aufweist.

2. Intermaxillares Befestigungssystem nach Anspruch 1, bei dem der Kopf (14) jedes Stiftes (10) in seiner Oberseite (16) eine Ausnehmung (18) aufweist, die zur Aufnahme der Spitze eines Schraubendrehers eingerichtet ist.

## Revendications

1. Système de fixation d'os comprenant une pluralité de taquets de fixation (10), chacun comprenant un arbre (12) se projetant à partir d'une tête élargie (14), l'arbre (12) étant fileté par vis et la tête ayant des surfaces supérieure et inférieure (16, 28) et une rainure annulaire (24) s'étendant autour dans une région entre lesdits surfaces supérieure et inférieure, et une bande, corde ou fil (20) qui est adapté pour l'entraînement autour des rainures des taquets respectifs (10) pour les lier ensemble quand, en fonctionnement, un ou plusieurs de tels taquets (10) sont fixés à chaque os ou partie d'os devant être joint, par le vissage de l'arbre (12) de celui-ci dans l'os ou la partie d'os, **caractérisé en ce que** le système est un système de fixation intermaxillaire et **en ce que** la tête (14) de chaque taquet (10) a seulement une simple rainure (24) de section transversale concave formée dedans.

2. Système de fixation intermaxillaire selon la revendication 1, **caractérisé en ce que** la tête (14) de chaque taquet (10) a un repli (18) dans sa surface supérieure (16), ledit repli étant adapté pour la réception d'une pointe de tournevis.
